# EUROPEAN PATENT APPLICATION

(11) **EP 4 652 990 A1**
(43) Date of publication of application: **26.11.2025**
(21) Application number: 24386059.0
(22) Date of filing: 24.05.2024
(51) Int. Cl.: A61K 9/20, A61K 9/28, A61K 31/216, A61K 31/41

(54) **A SOLID COMPOSITION PACKAGE OF VALSARTAN AND SACUBITRIL**

(71) Applicant: Genepharm S.A., 15351 Pallini (GR)
(72) Inventor: Mpenekis, Vasilis, 11475 Athens (GR); Xenogiorgis, Vasileios, 17676 Kallithea Athens (GR); Megalooikonomou, Kalliopi, 15343 Agia Paraskevi Athens (GR)
(74) Representative: HL Kempner PartG mbB

## Description

### FIELD OF THE INVENTION

The present invention relates to solid composition packages comprising amorphous valsartan disodium and crystalline sacubitril monosodium, and a process for the preparation of such solid composition packages.

### BACKGROUND OF THE INVENTION

Valsartan, which is chemically known as (2S)-3-methyl-2-[pentanoyl[[2'-(1H-tetrazol-5-yl)biphenyl-4-yl]methyl]amino]butanoic acid is represented structurally by Formula (I) below:

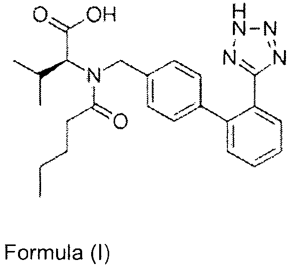

Valsartan is an angiotensin II receptor blocker medication commonly used to treat high blood pressure, heart failure and diabetic kidney disease.

Sacubitril, which is chemically known as 4-{[(2S,4R)-1-([1,1'-biphenyl]-4-yl)-5-ethoxy-4-methyl-5-oxopentan-2-yl]amino}-4-oxobutanoic acid is represented structurally by Formula (II) below:

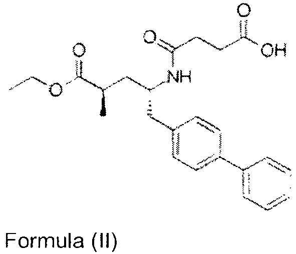

Sacubitril is an antihypertensive drug. Sacubitril is often used in the crystalline sacubitril monosodium form. Multiple crystalline forms of sacubitril monosodium exist, such as A, B, C, D and E. Valsartan is commonly used in combination with sacubitril to form a co-crystal tablet for the prevention of heart failure. Valsartan combined with sacubitril for the treatment of heart failure has been shown to significantly reduce cardiovascular mortality and hospitalisations. Entresto^{®} is a commercially available example of a combination of valsartan and sacubitril. Entresto^{®} contains a co-crystal consisting of valsartan disodium, sacubitril monosodium and 2.5 molecules water. The co-crystal has been designated as LCZ696. Synthesis of the co-crystal is described in WO2007/056546. As discussed in EP3766484B1, a potential drawback of the cocrystal of valsartan and sacubitril is the linked dissolution relationship between the two active ingredients.

Amorphous forms of valsartan disodium are very hygroscopic, causing handling and problems with processability due to becoming deliquescent and sticky. In particular, manufacturing problems can arise such as stickiness, poor flow characteristics, poor compressibility. The resultant compositions are often also hygroscopic, causing problems after storage such as insufficient amounts of the active ingredient. Additionally, amorphous valsartan disodium is generally less thermodynamically stable than crystalline valsartan disodium, and so can convert to the crystalline form over time.

EP4088715A1 describes a pharmaceutical formulation comprising a mixture of a first granular composition comprising valsartan disodium salt; and a second composition comprising or essentially consisting of sacubitril sodium salt. EP4088715A1 preferably uses wet granulation to prepare the granular composition. However, wet granulation often leads to a lack of stability of the formulation due to active pharmaceutical ingredients being moisture and heat sensitive. There is no disclosure or discussion within EP4088715A1 regarding the need for a moisture barrier, or about moisture control at all. In fact, this document is entirely silent about any possible effect of moisture on the stability of the granular composition. There is also no discussion or disclosure regarding the use of sacubitril monosodium crystalline form B.

EP3766484B1 describes a solid pharmaceutical dosage form comprising crystalline valsartan and crystalline sacubitril, which does not comprise a filler, a binder or disintegrants. EP3766484B1 focuses on developing a process that allows for lubricant to be used in the roller compaction or slugging process. The use of lubricants was previously avoided as they were often found to adversely affect dissolution characteristics of resulting tablets. However, lubricants play an important role in tablet manufacturing, as lubricants maintain flowability of powder by preventing powders from sticking together, which allows the manufacturing process to be more efficient. Lubricant can also increase the strength of tablets by reducing the friction between ingredients during compression. There is no discussion in EP3766484B1 regarding the long-term stability of the solid pharmaceutical dosage form comprising crystalline valsartan and crystalline sacubitril. EP3766484B1 describes a stable composition without the use of packaging. EP3766484B1 is also silent about the use of amorphous valsartan disodium and the effect the use of amorphous valsartan disodium may have on stability of the composition.

EP3694493A1 describes a tablet containing valsartan, sacubitril and a mesoporous inorganic stabiliser. The mesoporous inorganic stabiliser was found to be necessary within the tablet to provide stability and flowability. The valsartan and sacubitril were dry granulated together to form the tablet. EP3694493A1 therefore teaches that to provide a stable composition of valsartan and sacubitril, requires certain excipients, and valsartan and sacubitril need to be granulated together. There is therefore the need for a simpler procedure, that doesn't require the use of an inorganic stabiliser to address stability of the valsartan sacubitril composition.

It is therefore desirable to provide an alternative solid composition package comprising a solid composition comprising amorphous valsartan disodium and sacubitril monosodium. In particular, it is desirable to provide a solid composition package comprising a solid composition of amorphous valsartan disodium and crystalline sacubitril monosodium compositions that is stable (no polymorphic conversion, or crystallisation). It is also desirable to provide a solid composition package comprising a solid composition comprising amorphous valsartan disodium and crystalline sacubitril monosodium that is simple to manufacture.

### SUMMARY OF THE INVENTION

The present invention is defined in the appended claims.

In accordance with a first aspect, there is provided a solid composition package comprising: a solid composition comprising: valsartan disodium granules comprising amorphous valsartan disodium, at least one filler, at least one disintegrant, at least one lubricant and at least one glidant; sacubitril monosodium granules comprising sacubitril monosodium crystalline form B, at least one filler, at least one disintegrant, at least one lubricant and at least one glidant; and at least one extragranular excipient selected from fillers, binders, glidants and lubricants, wherein the solid composition is packaged in Alu/Alu blister packaging and/or a HDPE bottle with desiccant.

The inventors surprisingly found that a solid composition package according to the present invention exhibited stability. Examples of the present invention, where the solid composition of the solid composition package comprises granules of valsartan disodium and sacubitril monosodium that are separately formed and then combined, were shown to display stability over 9 months at 30°C and 75% relative humidity (RH). The present inventors found that providing a moisture barrier created by packaging the solid composition in Alu/Alu blister packaging and/or a HDPE bottle with desiccant improved the stability of the solid composition package. In particular, the present inventors found that providing a moisture barrier created by packaging the solid composition in Alu/Alu blister packaging and/or a HDPE bottle with desiccant prevented the formation of the valsartan sacubitril co-crystal. Advantageously, the inventors found there was no need for additional inorganic stabilizers, for example, mesoporous silica, to stabilise the composition.

In accordance with a second aspect, there is provided a process for the preparation a solid composition package according to the first aspect.

The inventors surprisingly found that a solid composition package prepared according to the present invention, where the valsartan disodium and sacubitril monosodium are granulated separately, exhibited improved stability compared to solid compositions prepared using conventional processing techniques, where valsartan disodium and sacubitril monosodium are granulated together. Examples of the present invention were shown to display stability over 9 months at 30°C and 75% RH when packaged in Alu/Alu blister packaging and/or HDPE bottle with desiccant.

In a third aspect, there is provided a solid composition comprising:
(a) valsartan disodium granules comprising amorphous valsartan disodium, at least one filler, at least one disintegrant, at least one lubricant and at least one glidant;
(b) sacubitril monosodium granules comprising sacubitril monosodium crystalline form B, at least one filler, at least one disintegrant, at least one lubricant and at least one glidant; and
(c) at least one extragranular excipient selected from fillers, binders, glidants and lubricants;
characterised in that the solid composition is stabilised by a moisture barrier created by packaging the solid composition in Alu/Alu blister packaging and/or a HDPE bottle with desiccant.

Certain embodiments of the present invention may provide one or more of the following advantages:
- desired stability of a solid composition comprising amorphous valsartan disodium and crystalline sacubitril monosodium;
- desired cost of a solid composition comprising amorphous valsartan disodium and crystalline sacubitril monosodium;
- desired ease of handling of a solid composition comprising amorphous valsartan disodium and crystalline sacubitril monosodium;
- desired ease of preparation of a solid composition comprising amorphous valsartan disodium and crystalline sacubitril monosodium;
- increased stability of a composition comprising amorphous valsartan disodium and crystalline sacubitril monosodium when packaged in Alu/Alu blister packaging and/or a HDPE bottle with desiccant.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

### BRIEF DESCRIPTION OF THE DRAWINGS

The invention will further be illustrated by reference to the following figures:
Figure 1: shows an XRD spectrum illustrating the stability of amorphous valsartan disodium and crystalline sacubitril monosodium tablets of Comparative Example 1, in a HDPE bottle with desiccant;
Figure 2: shows an XRD spectrum illustrating the stability of amorphous valsartan disodium and crystalline sacubitril monosodium tablets of Example 1 according to the present invention, in a HDPE bottle with desiccant;
Figure 3: shows an XRD spectrum illustrating the stability of amorphous valsartan disodium and crystalline sacubitril monosodium tablets of Example 1 according to the present invention, in Alu/Alu blister packaging;
Figure 4: shows an XRD spectrum illustrating the stability of amorphous valsartan disodium and crystalline sacubitril monosodium tablets of Example 2 according to the present invention, in Alu/Alu blister packaging.
Figure 5: shows an XRD spectrum illustrating the stability of amorphous valsartan disodium and crystalline sacubitril monosodium tablets of Example 3 according to the present invention, in Alu/Alu blister packaging;

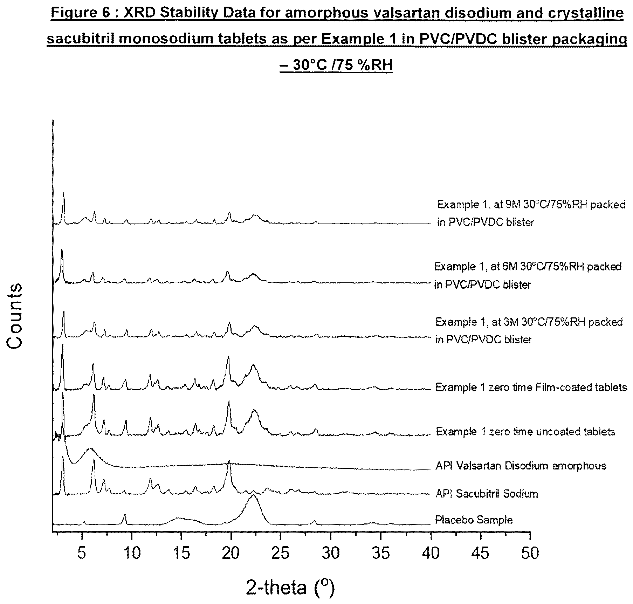

Figure 6: shows an XRD spectrum illustrating the stability of amorphous valsartan disodium and crystalline sacubitril monosodium tablets of Example 1 in PVC/PVDC blister packaging at 30°C /75 %RH; and

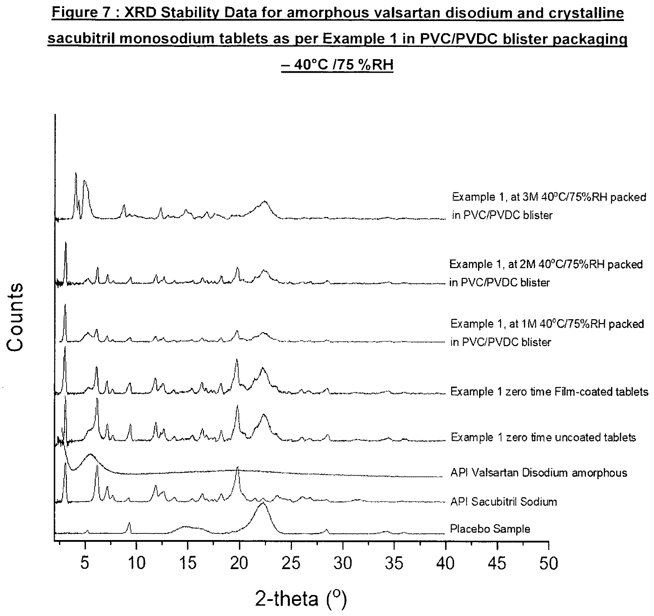

Figure 7: shows an XRD spectrum illustrating the stability of amorphous valsartan disodium and crystalline sacubitril monosodium tablets of Example 1 within PVC-PVDC packaging at 40°C /75 %RH.

It is understood that the following description and references to the figures concern exemplary embodiments of the present invention and shall not be limiting the scope of the claims.

### DETAILED DESCRIPTION

The present invention is based on the surprising finding that a solid composition package comprising a solid composition comprising separate granules of valsartan disodium and sacubitril monosodium that are then combined, was shown to display polymorphic stability over 9 months at 30°C and 75% RH when packaged in Alu/Alu blister packaging and/or a HDPE bottle with desiccant. The present inventors also surprisingly found a solid composition comprising separate granules of valsartan disodium and sacubitril monosodium that are then combined and compressed, was shown to display polymorphic stability over 6 months at 40°C and 75% RH when packaged in Alu/Alu blister packaging and/or a HDPE bottle with desiccant to form a solid composition package.

As used herein, the term "amorphous" refers to a solid material having no long range order in the position of its molecules. Amorphous solids cannot be defined by a finite unit cell. For example, unlike crystalline materials which exhibit strong Bragg diffraction, the X-Ray Diffraction patterns of amorphous materials are characterized by broad and diffuse peaks.

When ranges are used herein, all combinations and sub-combinations of ranges and specific embodiments therein are intended to be included.

As used herein, the term "about" when referring to a number or a numerical range means that the number or numerical range referred to is an approximation within experimental variability (or within statistical experimental error), and thus the number or numerical range may vary. Typical experimental variabilities may stem from, for example, changes and adjustments necessary during scale-up from laboratory experimental and manufacturing settings to large scale, GMP manufacturing conditions as is known to those familiar with the art of pharmaceutical development and manufacturing. Such changes can vary between 1% and 10% of the stated number or numerical range.

As used herein, the term "comprising" (and related terms such as "comprise" or "comprises" or "having" or "including") has an open meaning and therefore a pharmaceutical composition comprising described features may comprise additional components in addition to the described features.

Abbreviations used herein have their conventional meaning within the chemical and biological arts, unless otherwise indicated.

As used herein, the term "excipient" refers to an inactive ingredient in a composition.

As used herein, the term "diluent" or "filler" refers to an excipient that adds bulk to a composition.

As used herein, the term "disintegrant" refers to an excipient that promotes disintegration of a tablet/composition into smaller fragments in an aqueous environment.

As used herein, the term "surfactant" refers to an excipient that decreases surface or interfacial tension to impart compositions with enhanced solubility and/or wettability.

As used herein, the term "binder" refers to an excipient that improves cohesion, and plasticity of a composition.

As used herein, the term "treatment" refers to an approach for obtaining beneficial or desired results including, but not limited to, therapeutic benefit and/or a prophylactic benefit. The term "patient" refers to an animal, such as a mammal, for example a human.

As used herein, the term "intragranular" refers to a component added before a granulation process.

As used herein, the term "extragranular" refers to a component added after granulation and before a compression process.

As used herein, the term "solid composition package" refers to a solid composition in packaging. According to the present invention, a solid composition package refers to a solid composition comprising valsartan and sacubitril when packaged in Alu/Alu blister packaging and/or a HDPE bottle with desiccant.

The present invention provides a solid composition package comprising:
a solid composition comprising:
(a) valsartan disodium granules comprising amorphous valsartan disodium, at least one filler, at least one disintegrant, at least one lubricant and at least one glidant;
(b) sacubitril monosodium granules comprising sacubitril monosodium crystalline form B, at least one filler, at least one disintegrant, at least one lubricant and at least one glidant; and
(c) at least one extragranular excipient selected from fillers, binders, glidants and lubricants;
wherein the solid composition is packaged in Alu/Alu blister packaging and/or a HDPE bottle with desiccant.

The present inventors surprisingly found packaging the solid composition in Alu/Alu blister packaging and/or a HDPE bottle with desiccant increased the time period over which the solid composition was stable. In particular, the present inventors surprisingly found that a solid composition packaged in Alu/Alu blister packaging and/or a HDPE bottle with desiccant, according to the present invention, displayed better polymorphic stability, than the same solid composition packaged in PVC/PVDC blister packaging. The Alu/Alu blister packaging and/or a HDPE bottle with desiccant may act as a moisture-barrier.

In certain embodiments, the valsartan disodium granules and the sacubitril monosodium granules of the solid composition are dry granules.

In certain embodiments, the solid composition of valsartan disodium granules and sacubitril monosodium granules comprises a filler or diluent selected from silicified microcrystalline cellulose, microcrystalline cellulose, mannitol, starch, pregelatinized starch and lactose, or a combination thereof. In some embodiments, the lactose may be lactose anhydrous. In alternative embodiments, the lactose may be lactose monohydrate. In some embodiments, the solid composition comprises filler or diluent in an amount of about 20 wt.% to about 60 wt.%, for example, from about 20 wt.% to about 55 wt.%, from about 25 wt.% to about 50 wt.%, or from about 30 wt.% to about 40 wt.%. In certain embodiments, the solid composition comprises filler or diluent in an amount of about 30 wt.% to about 35 wt.%. For example, the solid composition may comprise filler or diluent in an amount of about 32 wt.%. In an embodiment, the solid composition of valsartan disodium granules and sacubitril monosodium granules comprises microcrystalline cellulose.

In certain embodiments, the solid composition of valsartan disodium granules and sacubitril monosodium granules comprises a disintegrant selected from pregelatinized starch, croscarmellose sodium and crospovidone, or a combination thereof. In some embodiments, the solid composition comprises disintegrant in an amount of about 1 wt.% to about 12.5 wt.%, for example, from about 2 wt.% to about 10 wt.%, from about 4 wt.% to about 9 wt.%, or from about 7.5 wt.% to about 8.5 wt.%. In certain embodiments, the solid composition comprises disintegrant in an amount from about 7 wt.% to about 9 wt.%. For example, the solid composition may comprise disintegrant in an amount of about 8 wt.%. In an embodiment, the solid composition of valsartan disodium granules and sacubitril monosodium granules comprises crospovidone. In an embodiment, the solid composition is free-from low substituted hydroxypropyl cellulose.

In certain embodiments, the solid composition of valsartan disodium granules and sacubitril monosodium granules comprises a glidant selected from colloidal silica, fumed silica, talc and magnesium carbonate, or a combination thereof. In some embodiments, the solid composition comprises glidant in an amount of about 0.5 wt.% to about 7.5 wt.%, for example, from about 1 wt.% to about 7 wt.%, from about 2 wt.% to about 6 wt.%, from about 3 wt.% to about 5 wt.%, or from about 3.5 wt.% to about 4.5 wt.%. In some embodiments, for example, the solid composition according to the present invention comprises a glidant in an amount of about 4 wt.%. In an embodiment, the solid composition of valsartan disodium granules and sacubitril monosodium granules comprises talc. In an embodiment, the solid composition of valsartan disodium granules and sacubitril monosodium granules comprises silica. In an embodiment, the solid composition of valsartan disodium granules and sacubitril monosodium granules comprises both silica and talc. In such an embodiment, the silica may be colloidal silica, fumed silica or a combination thereof.

In certain embodiments, the solid composition of valsartan disodium granules and sacubitril monosodium granules comprises a lubricant selected from magnesium stearate and sodium stearyl fumarate, or a combination thereof. In some embodiments, the solid composition of valsartan disodium granules and sacubitril monosodium granules comprises intraganular and extragranular lubricant. In some embodiments, the solid composition comprises lubricant in an amount of from about 0.1 wt.% to about 5 wt.%, for example, from about 0.5 wt.% to about 4.5 wt.%, or from about 1 wt.% to about 4 wt.%. In some embodiments, the solid composition comprises intragranular lubricant in an amount of from about 0.1 wt.% to about 2 wt.%, for example, from about 0.5 wt.% to about 1.5 wt.%, from about 0.65 wt.% to about 0.95 wt.%. For example, the solid composition according to the present invention comprises intragranular lubricant in an amount of about 0.8 wt.%. In some embodiments, the solid composition comprises extragranular lubricant in an amount of from about 0.1 wt.% to about 2 wt.%, for example, from about 0.5 wt.% to about 1.5 wt.%, from about 0.75 wt.% to about 1.25 wt.%. For example, the solid composition according to the present invention comprises extragranular lubricant in an amount of about 1 wt.%.

In certain embodiments, the solid composition of valsartan disodium granules and sacubitril monosodium granules comprises a binder is selected from pregelatinized starch, polymethacrylates, copovidone, povidone, maltose, starch, cellulose powder, crystalline cellulose, microcrystalline cellulose, silicified microcrystalline cellulose, cellulose derivatives such as hydroxymethyl cellulose, hydroxyethyl cellulose, hydroxypropyl cellulose, low-substituted hydroxypropyl cellulose, hydroxypropylmethylcellulose and combinations thereof.

In certain embodiments, the solid composition of is a tablet. In certain embodiments, the solid composition is a monolayered tablet.

In certain embodiments, the solid composition of valsartan disodium granules and sacubitril monosodium granules comprises:
(a) valsartan disodium in an amount of about 20 wt.% to about 30 wt.%;
(b) sacubitril monosodium in an amount of about 20 wt.% to about 30 wt.%;
(c) extragranular excipient in an amount of about 10 wt.% to about 15 wt.%;
wherein wt.% is based on the total weight of the solid composition.

In certain embodiments, the solid composition of valsartan disodium granules and sacubitril monosodium granules is coated. In specific embodiments, the solid composition is a coated tablet. In specific embodiments, the solid composition is a monolayer coated tablet.

In certain embodiments, the monolayer tablet composition of valsartan disodium granules and sacubitril monosodium granules is coated with a polymeric film-coating material. Numerous suitable polymeric film coating materials are known in the art. In certain embodiments, the polymeric film coating material is selected from a film-coating polymer such as polyvinyl alcohol and/or polyethylene glycol. In some embodiments, the polymeric film-coating polymer is selected from hydroxypropylmethylcellulose or hydroxypropylcellulose.

In certain embodiments, in addition to the polymeric film-coating polymer, the polymeric film coating material further comprises a plasticizer. In some embodiments, the plasticizer is selected from propylene glycol, and optionally a pigment such as titanium dioxide. In some embodiments, the composition of the polymeric film-coating material comprises Hypromellose substitution type 2910, Titanium dioxide (E171), Talc and Macrogol (6000). The composition of the polymeric film-coating material may further comprise Iron oxide red (E172), Iron oxide black (E172), Iron oxide yellow (E172), or combinations thereof.

In some embodiments, the coating may be selected from Opadry PINK 00F240016, Opadry PINK 00F240015 or Opadry YELLOW 00F220016.

In certain embodiments, the polymeric film coating material may also include an anti-adhesive. In some embodiments, the anti-adhesive is talc.

The term stable as defined herein refers to a composition that that maintains its polymorphic state, and does not transition from crystalline to amorphous, or convert to the Sacubitril Valsartan cocrystal. The stability may be measured by suitable methods such as X-ray diffraction (XRD), dissolution and High-Performance Liquid Chromatography (HPLC). For example, the amorphous nature and active purity of the solid composition, of the solid composition granules, is retained at about 40 °C and about 75% relative humidity for at least about 6 months when packed in Alu/Alu blister packaging and/or a HDPE bottle with desiccant.

In certain embodiments, the solid composition of valsartan disodium granules and sacubitril monosodium granules is stable for at least about 1 month, at least about 2 months, at least about 3 months, at least about 4 months, at least about 5 months, at least about 6 months, at least about 7 months, at least about 8 months, or at least about 9 months at about 30 °C and about 75% relative humidity when packed in Alu/Alu blister packaging and/or a HDPE bottle with desiccant. In certain embodiments, the sacubitril monosodium crystalline form B in the solid composition is stable at 30°C and 75% RH for at least 3 months when packed in Alu/Alu blister packaging and/or a HDPE bottle with desiccants, for example, the sacubitril monosodium crystalline form B in the solid composition is stable at 30°C and 75% RH for at least 4 months, at least about 5 months, at least about 6 months, at least about 7 months, at least about 8 months, or at least about 9 months at 30°C and 75% RH, when packed in Alu/Alu blister packaging and/or a HDPE bottle with desiccants.

In certain embodiments, the solid composition of valsartan disodium granules and sacubitril monosodium granules is stable for at least about 1 month, at least about 2 months, at least about 3 months, at least about 4 months, at least about 5 months, at least about 6 months, at least about 7 months or at least about 8 months, at about 40 °C and about 75% relative humidity when packed in Alu/Alu blister packaging and/or a HDPE bottle with desiccant.

In embodiments, the sacubitril monosodium crystalline form B of the solid composition package is stable at 30°C and 75% RH for at least about 3 months, for example, for at least about 4 months, at least about 5 months, at least about 6 months at least about 7 months, at least about 8 months, or at least about 9 months.

In embodiments, the sacubitril monosodium crystalline form B of the solid composition package is stable at 40°C and 75% RH for at least about 3 months, for example, for at least about 4 months, at least about 5 months, at least about 6 months, at least about 7 months or at least about 8 months.

In certain embodiments, the solid composition is free from crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate characterized by X-ray powder diffraction interlattice plane intervals: d in [Å] (+/- 0.1 Å): 21.2(s), 17.0(w), 7.1 (s), 5.2(w), 4.7(w), 4.6(w), 4.2(w), 3.5(w), 3.3(w).

The term "free from crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate" as used herein refers to compositions where crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-yimethyl}amino)butyrate] hemipentahydrate has not been formed in the composition to achieve a particular effect.

For example, "free from crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate" means that crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate is present in the composition in an amount of less than about 0.5 wt.%, or less than about 0.3 wt.%, or less than about 0.2 wt.%, or less than about 0.1 wt.%, or less than about 0.05 wt.%, or less than about 0.01 wt.%, or less than about 0.005 wt.% based on the total weight of the solid composition. The presence of crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate may be identified by X-ray diffraction. For example, X-ray diffraction using a Bruker D2 Phaser or Bruker D2 Phaser XE-T Edition Instrument with a Cu Kα (λ=1.54 Å) source and a LynxEye detector.

In certain embodiments, the solid composition is free from crystalline trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate characterized by X-ray powder diffraction interlattice plane intervals: d in [Å] (+/- 0.1 Å): 21.2(s), 17.0(w), 7.1 (s), 5.2(w), 4.7(w), 4.6(w), 4.2(w), 3.5(w), 3.3(w) for at least 6 months, when stored at 30⁰C/75% RH in Alu/Alu blister packaging and/or a HDPE bottle with desiccant. For example, in certain embodiments the solid composition is free from crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate characterized by X-ray powder diffraction interlattice plane intervals: d in [Å] (+/- 0.1 Å): 21.2(s), 17.0(w), 7.1 (s), 5.2(w), 4.7(w), 4.6(w), 4.2(w), 3.5(w), 3.3(w) for at least 7 months, or at least 8 months, or at least 9 months, when stored at 30⁰C/75% RH in Alu/Alu blister packaging and/or a HDPE bottle with desiccant.

In certain embodiments, the solid composition is free from mesoporous inorganic stabilisers.

The term "free from mesoporous inorganic stabilisers" as used herein refers to compositions where a mesoporous inorganic stabiliser has not been added in the composition to achieve a particular effect. For example, "free from mesoporous inorganic stabilisers" means that mesoporous inorganic stabilisers are present in the composition in an amount of less than about 0.5 wt.%, or less than about 0.3 wt.%, or less than about 0.2 wt.%, or less than about 0.1 wt.%, or less than about 0.05 wt.%, or less than about 0.01 wt.%, or less than about 0.005 wt.% based on the total weight of the solid composition.

In certain embodiments, the tablets formed from the solid composition of the present invention comprise up to about 97 mg of sacubitril and 103 mg of valsartan in a single dose. For example, the tablets formed from the solid composition of the present invention may comprise up to about 49 mg of sacubitril and about 51 mg of valsartan in a single dose or about 24 mg of sacubitril and about 26 mg of valsartan in a single dose. The solid compositions of sacubitril and valsartan are suitable for oral administration of sacubitril and valsartan as a treatment for a patient.

In certain embodiments, the solid composition is an immediate release composition.

As used herein, the term "immediate release" generally refers to a composition that is developed to dissolve without delaying or prolonging dissolution or absorption of the drug.

In certain embodiments, the immediate release composition according to the present invention has less than about 5 wt.% coating based on the total weight of the solid composition, for example, less than about 4 wt.% coating, less than about 3 wt.% coating, less than about 2 wt.% coating, less than about 1 wt.%, or less than about 0.5 wt.% coating based on the total weight of the solid composition.

In an embodiment, the solid composition may comprise:
(a) valsartan disodium granules comprising amorphous valsartan disodium, at least one filler, at least one disintegrant, at least one lubricant and at least one glidant;
(b) sacubitril monosodium granules comprising sacubitril monosodium crystalline form B, at least one filler, at least one disintegrant, at least one lubricant and at least one glidant; and
(c) at least one extragranular excipient selected from fillers, binders, glidants and lubricants;
characterised in that the solid composition is stabilised by a moisture barrier created by packaging the solid composition in Alu/Alu blister packaging and/or a HDPE bottle with desiccant.

In certain embodiments, a process for the preparation of a solid composition of the present invention comprises:
(i) preparing a valsartan disodium blend comprising valsartan disodium amorphous and at least one filler, at least one disintegrant, at least one lubricant and at least one glidant;
(ii) dry granulating the valsartan disodium blend to form valsartan disodium granules;
(iii) preparing a sacubitril monosodium blend comprising sacubitril monosodium crystalline form B and at least one filler, at least one disintegrant, at least one lubricant and at least one glidant
(iv) dry granulating the sacubitril monosodium blend to form sacubitril monosodium granules;
(v) mixing the valsartan disodium granules and the sacubitril monosodium granules with extragranular excipients selected from fillers, binders, glidants and lubricants;
(vi) compressing the mixture;
(vii) coating the compressed mixture; and
(viii) packaging the solid composition in Alu/Alu blister packaging and/or a HDPE bottle with desiccant.

In other words, the solid composition package is prepared by first preparing a solid composition of separate valsartan disodium and sacubitril monosodium granules by dry granulation. The valsartan disodium and sacubitril monosodium granules are then mixed together with extragranular excipients compressed and coated. Oncecoated, the solid composition is packaged in Alu/Alu blister packaging and/or a HDPE bottle with desiccant to form the solid composition package. In a particular embodiment, the valsartan disodium and sacubitril monosodium granule mixture is mixed with extragranular excipients and compressed into a tablet and coated.

In certain embodiments, the solid composition is prepared using roller compaction.

Roller compaction is beneficially a continuous process and so can lead to a production time reduction, and the scale-up may be considered easier than other dry granulation methods, therefore, production costs can be reduced, whilst overall production efficiency is improved. Furthermore, another advantage of roller compaction is that the bulk density of the obtained granule may be increased and/or easily maintained.

The roller compaction may comprise more than one roller compaction cycle, for example, the valsartan disodium granules and sacubitril monosodium granules may separately be passed through a roller compactor, sieved, and then passed through a roller compactor again. The sieving may involve the granules being pass through a cone mill or through a hand sieve. For example, the dry granules may be passed through a 30-mesh sieve or passed through a Cone mill at a rotation speed of 1195 rpm and through a G-type sieve of size 1270 µm with a round rotating impeller.

In certain embodiments, the composition may have one or more of the following effects:
- desired stability of a composition comprising amorphous valsartan disodium and crystalline sacubitril monosodium;
- desired cost of a composition comprising amorphous valsartan disodium and crystalline sacubitril monosodium;
- desired ease of handling of a composition comprising amorphous valsartan disodium and crystalline sacubitril monosodium;
- desired ease of preparation of a solid composition of amorphous valsartan disodium and crystalline sacubitril monosodium;
- increased stability of a composition of amorphous valsartan disodium and crystalline sacubitril monosodium within Alu/Alu blister packaging and/or a HDPE bottle with desiccant.

### EXAMPLES

The following illustrates examples of the solid compositions and solid composition packages of amorphous valsartan disodium and crystalline sacubitril monosodium, and related aspects described herein. Thus, these examples should not be considered to restrict the present disclosure, but are merely in place to teach how to carry out the present disclosure.

### Section A: Compositions and their methods of production

To test manufacturing suitability and feasibility, four examples were screened. Three examples were prepared according to the present invention. One Comparative Example was used. Comparative Example 1 does not comprise separate valsartan disodium granules and sacubitril monosodium granules, Comparative Example 1 comprises valsartan disodium and sacubitril monosodium that have been granulated together.

The compositions of Examples 1 to 3, comprising 97 mg of sacubitril and 103 mg of valsartan, 24 mg of sacubitril and 26 mg of valsartan, and 49 mg of sacubitril and 51 mg of valsartan respectively, and Comparative Example 1 comprising 97 mg of sacubitril and 103 mg of valsartan, are shown below in Table 1.

**Table 1 - Composition of Examples 1 to 3 and Comparative Example 1 as prepared**

| | Comparative Example 1 (97/103 mg) | Example 1 (97/103 mg) | Example 2 (24/26 mg) | Example 3 (49/51 mg) |
|---|---|---|---|---|
| **Components** | **% by weight** | **% by weight** | **% by weight** | **% by weight** |
| **Intragranular** | **Intragranular** | **Intragranular** | **Intragranular** | **Intragranular** |
| Sacubitril Sodium crystalline form B | 25.6 | 25.6 | 25.6 | 25.6 |
| Microcrystalline Cellulose PH102 (Ph. Eur.) | 33.0 | 13.8 | 13.8 | 13.8 |
| L-HPC | 0.6 | - | - | - |
| Crospovidone, Type A (Ph. Eur.) | 1.0 | 1.0 | 1.0 | 1.0 |
| Colloidal Silicon dioxide (Ph. Eur.) | 1.0 | 0.5 | 0.5 | 0.5 |
| Talc (Ph. Eur.) | 2.0 | 1.0 | 1.0 | 1.0 |
| Magnesium Stearate (Ph. Eur.) | 1.0 | 0.4 | 0.4 | 0.4 |

| **Intragranular** | **-** | **Intragranular** | **Intragranular** | **Intragranular** |
|---|---|---|---|---|
| Valsartan Disodium amorphous | 28.3 | 28.3 | 28.3 | 28.3 |
| Microcrystalline Cellulose PH102 (Ph. Eur.) | *included above* | 10.0 | 10.0 | 10.0 |
| Crospovidone, Type A (Ph. Eur.) | *included above* | 4.0 | 4.0 | 4.0 |
| Colloidal Silicon dioxide (Ph. Eur.) | *included above* | 0.5 | 0.5 | 0.5 |
| Talc (Ph. Eur.) | *included above* | 1.0 | 1.0 | 1.0 |
| Magnesium Stearate (Ph. Eur.) | *included above* | 0.4 | 0.4 | 0.4 |

| **Extragranular** | **Extragranular** | **Extragranular** | **Extragranular** | **Extragranular** |
|---|---|---|---|---|
| Microcrystalline Cellulose PH102 (Ph. Eur.) | - | 8.5 | 8.5 | 8.5 |
| Crospovidone, Type A (Ph. Eur.) | 5.0 | 3.0 | 3.0 | 3.0 |
| Colloidal Silicon dioxide (Ph. Eur.) | 1.0 | 1.0 | 1.0 | 1.0 |
| Magnesium Stearate (Ph. Eur.) | 1.5 | 1.0 | 1.0 | 1.0 |

| **Coating** | **Coating** | **Coating** | **Coating** | **Coating** |
|---|---|---|---|---|
| Opadry PINK 00F240016 | 3.0 | 3.0 | - | - |
| Opadry PINK 00F240015 | - | - | 3.0 | - |
| Opadry YELLOW 00F220016 | - | - | - | 3.0 |
| **Coated Tablet Weight** | 103.00 | 103.00 | 103.00 | 103.00 |

### Method of preparation for Examples 1 to 3

Examples 1 to 3 according to the present invention were prepared according to the following steps:

### Step 1a: Formation of Sacubitril Granules

- Each intragranular component for the Sacubitril Granules was sieved through a 30-mesh sieve and mixed together in a drum mixer at 40 rpm to form Blend A.
- The Magnesium Stearate lubricant was sieved through a 40-60-mesh sieve and mixed with Blend A to form a lubricated blend, Blend B.
- Blend B was then dry granulated through a roller compactor according to specific parameters, detailed in Table 2 below, in which two roller compaction cycles were used, to form Sacubitril Blend dry granules.
- Between the two roller compactor cycles the Blend B was crushed and passed through a 30-mesh sieve or passed through a Cone mill at a rotation speed of 1195 rpm and through a G-type sieve of size 1270 µm with a round rotating impeller.

### Step 1b: Formation of Valsartan Granules

- Each intragranular component for the Valsartan Granules was sieved through a 30-mesh sieve and mixed together in a drum mixer at 40 rpm to form Blend X.
- The Magnesium Stearate lubricant was sieved through a 40-60-mesh sieve and mixed with Blend X to form a lubricated blend, Blend Y.
- Blend Y was then dry granulated through a roller compactor according to specific parameters, detailed in Table 2 below. Two roller compaction cycles were used to form Valsartan Blend dry granules.
- Between the two roller compactor cycles, Blend Y was crushed and passed through a 30-mesh sieve.

### Step 2a: Sizing of Sacubitril Blend dry granules

- The Sacubitril Blend dry granules resulting from Step 1a were then crushed and passed through a 30-mesh sieve or a Cone mill at a rotation speed of 1195 rpm and through a G-type sieve of size 1270 µm with round rotating impeller, and mixed to form Blend C.

### Step 2b: Sizing of Valsartan Blend dry granules

- The Valsartan Blend dry granules resulting from Step 1b were then crushed and passed through a 30-mesh sieve or a Cone mill at a rotation speed of 1195 rpm and through a G-type sieve of size 1270 µm with round rotating impeller, and mixed to form Blend Z.

### Step 3: Mixing to form Blend H

- Each extragranular component was sieved through a 30-mesh sieve.
- The sieved extragranular components were mixed in a drum mixer with Blend C and Blend Z for 30 min, and at 40 rpm rotation speed to form blend H.

### Step 4: Lubrication

- The Magnesium Stearate lubricant was sieved through a 40-60-mesh sieve, and added mixed with Blend H for 1-2 minutes at 40 rpm rotation speed, to form lubricated Blend H.

### Step 5: Compression

- Lubricated blend H from Step 4 was compressed to form core tablets.

### Step 6: Coating

- The compressed core tablets of Example 1 (97/103 mg) were coated with Opadry PINK 00F240016.
- The compressed core tablets of Example 2 (24/26 mg) were coated with Opadry PINK 00F240015.
- The compressed core tablets of Example 3 (49/51 mg) were coated with Opadry YELLOW 00F220016.

### Table 2 - Roller compaction parameters

| **Parameter** | **Range** |
|---|---|
| Main Roll Speed (rpm) | 2-18 |
| Feed Screw Speed (rpm) | 10 - 150 |
| Roller Distance (mm) | 0.3 - 4.0 |
| Load on Main Roller (kg) | 0 - 250 |
| Number of Rollers | 2 |

### Method of preparation of Comparative Example 1

Comparative Example 1 was prepared according to the following steps:

### Step 1: Formation of Valsartan and Sacubitril Dry Granules

- The intragranular components (except for Magnesium Stearate) were sieved through a 30-mesh sieve and mixed to form Blend A.
- Magnesium Stearate was sieved through a 40-60-mesh sieve, and mixed with Blend A to form a lubricated blend, blend B.
- Blend B was then dry granulated through a roller compactor according to specific parameters, detailed in Table 3 below to form Valsartan and Sacubitril dry granules. Two roller compaction cycles were used to form Valsartan and Sacubitril dry granules.
- Between the two roller compactor cycles, Blend B was crushed and passed through a 30-mesh sieve.

### Step 2: Sizing of Valsartan and Sacubitril Blend

- The dry granules resulting from Step 1 were then crushed and passed through a 30-mesh sieve, and mixed to form Blend C.

### Step 3: Mixing to form Blend D

- Each extragranular component was sieved through a 30-mesh sieve.
- The sieved extragranular components were mixed in a drum mixer with Blend C for 30 min, and at 40 rpm speed to form blend D.

### Step 4: Lubrication

- The Magnesium Stearate lubricant was sieved through a 40-60-mesh sieve, and mixed with Blend D for 1-2 minutes at 40 rpm speed, to form lubricated Blend D.

### Step 5: Compression

- Lubricated blend D from Step 4 was compressed to form core tablets.

### Step 6: Coating

- The compressed core tablets from Step 5 were coated with Opadry PINK 00F240016.

**Table 3 - Roller compaction parameters**

| **Parameter** | **Range** |
|---|---|
| Main Roll Speed (rpm) | 2-18 |
| Feed Screw Speed (rpm) | 10 - 150 |
| Roller Distance (mm) | 0.3-4.0 |
| Load on Main Roller (kg) | 0 - 250 |
| Number of Rollers | 2 |

### SECTION B: Stability data for Examples 1 to 3 and Comparative Example 1

Stability testing was carried out based on the tablets of Examples 1 to 3 and Comparative Example 1.

The polymorphic stability of Examples 1 to 3 and Comparative Example 1 was investigated using X-ray diffraction. The polymorphic stability of tablets of Example 1 and Comparative Example 1 stored in a HDPE bottle with desiccant, was tested. The polymorphic stability of Examples 1 to 3 packaged in Alu/Alu packaging was also investigated. The conditions the tablets of Examples 1 to 3 and Comparative Example 1 were stored in, and the physical stability of the solid composition packages is summarised below in Tables 4 and 5. Stable refers to a composition that maintains its polymorphic state, and does not transition from crystalline to amorphous or convert to the Sacubitril Valsartan cocrystal.

**Table 4 - Stability of solid composition packages of Comparative Example 1 and Example 1 stored in a HDPE bottle with desiccant**

| **Time** | **Temperature (°C)** | **Relative Humidity (%)** | **Comparative Example 1** | **Example 1 (97/103 mg)** |
|---|---|---|---|---|
| 3 months | 25 | 60 | Stable | Stable |
| 3 months | 40 | 75 | Unstable | Stable |
| 5 months | 25 | 60 | Stable | Stable |
| 5 months | 40 | 75 | Unstable | Stable |
| 6 months | 25 | 60 | Stable | Stable |
| 6 months | 40 | 75 | Unstable | Stable |
| 7 months | 25 | 60 | Unstable | Stable |
| 7 months | 40 | 75 | Unstable | Stable |
| 8 months | 40 | 75 | Unstable | Stable |
| 9 months | 30 | 75 | Unstable | Stable |

**Table 5 - Stability of solid composition packages of Examples 1 to 3 stored in Alu/Alu packaging**

| **Time** | **Temperature (°C)** | **Relative Humidity (%)** | **Example 1 (97/103 mg)** | **Example 2 (24/26 mg)** | **Example 3 (49/51 mg)** |
|---|---|---|---|---|---|
| 3 months | 40 | 75 | Stable | Stable | Stable |
| 6 months | 40 | 75 | Stable | Stable | Stable |
| 9 months | 30 | 75 | Stable | Stable | Stable |

Placebo samples were also prepared with the same qualitative and quantitative (adjusted to 100% final ratio) compositions as per Comparative Example 1 and Examples 1 to 3, without the use of the active ingredients. Sacubitril Sodium API and Valsartan Disodium API were also used as comparative examples for XRD analysis.

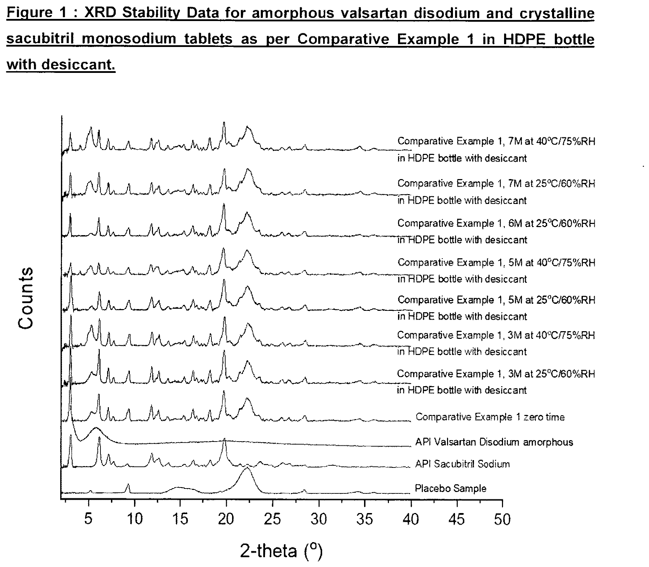

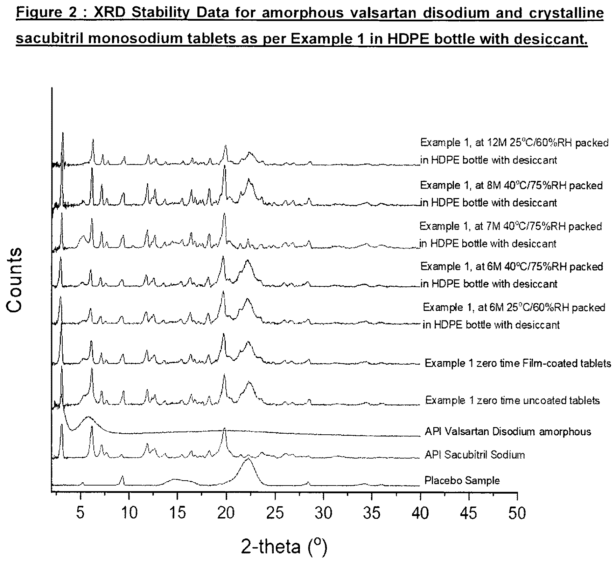

As shown in Figure 1, when stored in a HDPE bottle with desiccant, Example 1 maintains its crystallinity and shows good polymorphic stability up to 9 months. Figure 1 also demonstrates the composition of the present invention is stable and does not convert to Sacubitril Valsartan cocrystal, as seen in the prior art. Figure 2 shows that Comparative Example 1, in which the valsartan disodium and sacubitril monosodium were granulated together showed poor polymorphic stability up to 3 months at 40 °C and 75% RH.

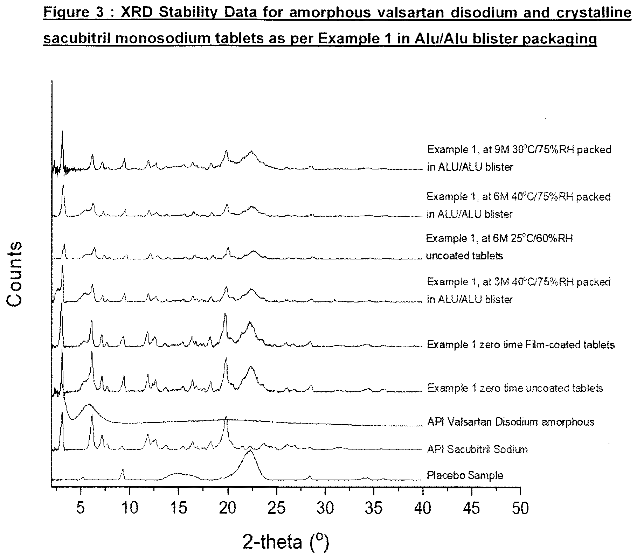

As shown in Figures 3 to 5, Examples 1, 2 and 3 maintain crystallinity and show good polymorphic stability up to 9 months at 30 °C and 75% relative humidity when stored in Alu/Alu packaging. Figures 3 to 5 also demonstrate the compositions of the present invention are stable and do not convert to the Sacubitril Valsartan cocrystal seen in the prior art.

XRD was performed according to the methodology of Chapter 2.9.33 of the European Pharmacopoeia, 11th Edition (2023), using a Bruker D2 Phaser Instrument, with a Cu Kα (λ=1.54 Å) source and a LynxEye detector.

The stability tests indicate that the tablets of Examples 1 to 3 display good polymorphic stability. The stability of compositions according to the present invention, where separate valsartan disodium and sacubitril monosodium granules are first formed, was found by the inventors to be improved compared to compositions where the valsartan disodium and sacubitril monosodium are granulated together.

### SECTION C: Stability influence of solid composition package

The effect of the Alu/Alu blister packaging and/or HDPE bottle with desiccant packaging on the stability of the solid composition, and therefore the solid composition package was also investigated. To do so, a different type of packaging to the Alu/Alu blister packaging and a HDPE bottle with desiccant used for the stability testing above was tested. Polyvinyl chloride and Polyvinylidene chloride (PVC-PVDC) blister packaging is a commonly used barrier film for pharmaceutical blister packs. Tablets of Example 1 were packaged in PVC-PVDC blister packaging and subjected to two separate packaging conditions. The tablets were kept in the PVC-PVDC blister packaging at 30°C/75% RH and 40°C/75% RH.

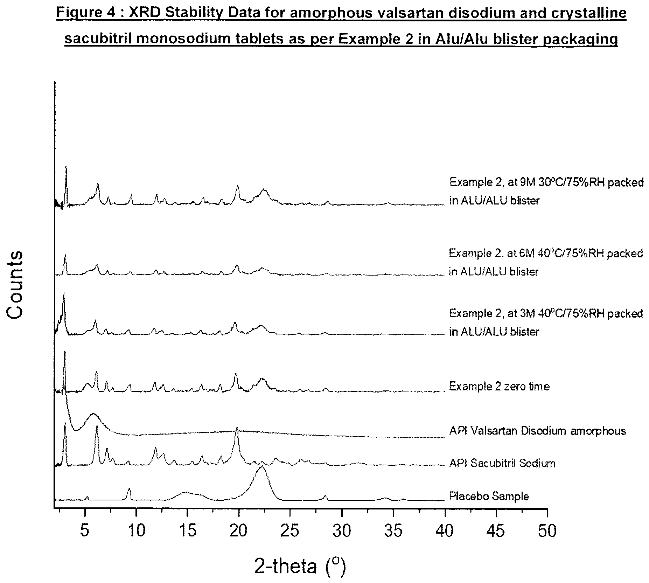

Figure 4 shows the stability of Example 1 within PVC/PVDC blister packaging at 30°C/75% RH. After 9 M at 30°C/75% RH, the polymorphic stability of Example 1 was reduced. An additional peak was formed at 4.1° theta. This peak may be due to cocrystal formation of the sacubitril and valsartan.

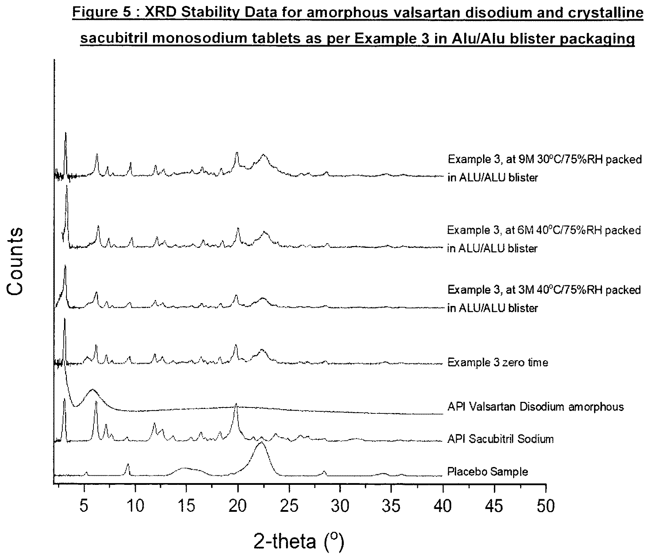

Figure 5 shows the stability of Example 1 within PVC/PVDC blister packaging at 40°C/75% RH. After 3 M at 40°C/75% RH, the polymorphic stability of Example 1 was reduced. Additional peaks were formed at 4.9° and 4.2° theta. These peaks may be due to co-crystal formation of the sacubitril and valsartan.

The XRD data indicates that the solid composition package, using Alu/Alu blister packaging and/or a HDPE bottle with desiccant, according to the present invention displayed better polymorphic stability, than when an identical solid composition was packaged in PVC/PVDC blister packaging. The present inventors found that the process of forming separate valsartan disodium and sacubitril monosodium that are then mixed together, alongside the use of Alu/Alu blister packaging and/or a HDPE bottle with desiccant resulted in tablets showing advantageous stability at 30°C and 75% relative humidity for up to 9 months. The present inventors also found that the process of forming separate valsartan disodium and sacubitril monosodium granules that are then mixed together, alongside the use of Alu/Alu blister packaging and/or a HDPE bottle with desiccant resulted in tablets showing advantageous stability at 40°C and 75% relative humidity for up to 6 months. The increased stability shown when the solid composition is packaged in Alu/Alu blister packaging and/or a HDPE bottle with desiccant is particularly advantageous as it not only provides a longer shelf-life for the solid composition, but also ensures that patients consuming the solid composition are taking effective regimens.

## Claims

1. A solid composition package comprising:
a solid composition comprising:
(a) valsartan disodium granules comprising amorphous valsartan disodium, at least one filler, at least one disintegrant, at least one lubricant and at least one glidant;
(b) sacubitril monosodium granules comprising sacubitril monosodium crystalline form B, at least one filler, at least one disintegrant, at least one lubricant and at least one glidant; and
(c) at least one extragranular excipient selected from fillers, binders, glidants and lubricants;
wherein the solid composition is packaged in Alu/Alu blister packaging and/or a HDPE bottle with desiccant.

2. A solid composition package as claimed in claim 1, wherein the solid composition is a tablet.

3. A solid composition package as claimed in claim 1 or claim 2, wherein the solid composition is a monolayered tablet.

4. A solid composition package as claimed in any one of the preceding claims, wherein the solid composition is an immediate release composition.

5. A solid composition package as claimed in any one of the preceding claims, wherein the valsartan disodium granules and the sacubitril monosodium granules of the solid composition are dry granules.

6. A solid composition package as claimed in any one of the preceding claims, wherein the sacubitril monosodium crystalline form B is stable at 30°C and 75% RH for at least 3 months.

7. A solid composition package as claimed in any one of the preceding claims, wherein the sacubitril monosodium crystalline form B is stable at 30°C and 75% RH for at least 6 months.

8. A solid composition package as claimed any one of the preceding claims, wherein the sacubitril monosodium crystalline form B is stable at 40°C and 75% RH for at least 6 months.

9. A solid composition package as claimed in any one of the preceding claims, wherein the solid composition is free from crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate, **characterized by** X-ray powder diffraction interlattice plane intervals: d in [Å] (+/- 0.1 Å): 21.2(s), 17.0(w), 7.1 (s), 5.2(w), 4.7(w), 4.6(w), 4.2(w), 3.5(w), 3.3(w).

10. A solid composition package as claimed in any one of the preceding claims, wherein the solid composition is free from crystalline trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate **characterized by** X-ray powder diffraction interlattice plane intervals: d in [Å] (+/- 0.1 Å): 21.2(s), 17.0(w), 7.1 (s), 5.2(w), 4.7(w), 4.6(w), 4.2(w), 3.5(w), 3.3(w) when stored at 30⁰C/75% RH for at least 6 months.

11. A solid composition package as claimed in any one of the preceding claims, wherein the solid composition comprises:
(a) valsartan disodium in an amount of about 20 wt.% to about 30 wt.%;
(b) sacubitril monosodium in an amount of about 20 wt.% to about 30 wt.%;
(c) extragranular excipient in an amount of about 10 wt.% to about 15 wt.%;
wherein wt.% is based on the total weight of the solid composition.

12. A solid composition package according to any one of the preceding claims, wherein the solid composition is free from mesoporous inorganic stabilisers.

13. A solid composition package according to any one of the preceding claims, wherein the solid composition is coated.

14. A process for the preparation of a solid composition package as claimed in any one of the preceding claims, the process comprising:
(i) preparing a valsartan disodium blend comprising valsartan disodium amorphous and at least one filler, at least one disintegrant, at least one lubricant and at least one glidant
(ii) dry granulating the valsartan disodium blend to form valsartan disodium granules;
(iii) preparing a sacubitril monosodium blend comprising sacubitril monosodium crystalline form B and at least one filler, at least one disintegrant, at least one lubricant and at least one glidant
(iv) dry granulating the sacubitril monosodium blend to form sacubitril monosodium granules;
(v) mixing the valsartan disodium granules and the sacubitril monosodium granules with extragranular excipients selected from fillers, binders, glidants and lubricants;
(vi) compressing the mixture;
(vii) coating the compressed mixture; and
(viii) packaging the solid composition in Alu/Alu blister packaging and/or a HDPE bottle with desiccant.

15. A process for the preparation of a solid composition as claimed in claim 14, wherein the dry granulating is performed using roller compaction.
